(19) Europäisches Patentamt

European Patent Office

Office européen des brevets

(11) EP 0 630 220 B1

(12) EUROPÄISCHE PATENTSCHRIFT

(45) Veröffentlichungstag und Bekanntmachung des Hinweises auf die Patenterteilung:
**23.04.1997 Patentblatt 1997/17**

(21) Anmeldenummer: 93903171.2

(22) Anmeldetag: **15.02.1993**

(51) Int. Cl.⁶: $A61F\ 7/00$, $A61B\ 19/00$

(86) Internationale Anmeldenummer:
**PCT/DE93/00126**

(87) Internationale Veröffentlichungsnummer:
**WO 93/17646 (16.09.1993 Gazette 1993/22)**

(54) **VERFAHREN UND ANORDNUNG ZUR THERAPIE VON GEWEBE MIT ULTRASCHALL**

ULTRASOUND TISSUE THERAPY PROCESS AND DEVICE

PROCEDE ET DISPOSITIF DE THERAPIE DES TISSUS PAR ULTRASONS

(84) Benannte Vertragsstaaten:
**DE FR GB IT**

(30) Priorität: **10.03.1992 DE 4207463**

(43) Veröffentlichungstag der Anmeldung:
**28.12.1994 Patentblatt 1994/52**

(73) Patentinhaber: **SIEMENS AKTIENGESELLSCHAFT**
**80333 München (DE)**

(72) Erfinder:
- **OPPELT, Arnulf**
  **D-8521 Spardorf (DE)**
- **REICHENBERGER, Helmut**
  **D-8501 Eckental (DE)**

(56) Entgegenhaltungen:
| | |
|---|---|
| **EP-A- 0 170 416** | **EP-A- 0 332 871** |
| **WO-A-89/06512** | **DE-A- 3 150 513** |
| **GB-A- 2 113 099** | **US-A- 4 343 301** |
| **US-A- 4 893 624** | **US-A- 4 938 216** |

**Beschreibung**

Die Erfindung betrifft eine Therapieeinrichtung zur Behandlung von Gewebe im Körper eines Lebewesens, aufweisend wenigstens einen therapeutischen Ultraschallwandler, der einen therapeutischen Wirkbereich aufweisenden Ultraschall aussendet, Mittel zum Einkoppeln des mittels des Ultraschallwandlers erzeugten Ultraschalls in den Körper des Lebewesens, Mittel zum Verlagern des therapeutischen Wirkbereiches des Ultraschalls und des Körpers des Lebewesens relativ zueinander.

Derartige Therapieeinrichtungen werden beispielsweise zur Behandlung der benignen Prostatahyperplasie (BPH = gutartige Wucherung der Prostata mit fortschreitender Verengung der Urethra (Harnröhre), tritt bei Männern etwa ab dem 50. Lebensjahr mit dem Alter zunehmend auf). Für die Therapie der BPH werden als Alternative zu den klassischen Verfahren der Operation und transurethralen Resektion in zunehmendem Maß weniger invasive Verfahren vorgeschlagen und untersucht. Im einzelnen zu nennen sind:

- Hyperthermie der Prostata durch Erwärmung bis 45°C mit Mikrowellen über einen rektalen Applikator (US 4 601 296) oder einen urethralen Applikator (US 4 967 765),

- in abgewandelter Form (höhere Temperatur) als Thermotherapie in Verbindung mit Ultraschall-Bildgebung und Kühlung der Urethra (EP 0 459 535),

- transrektale Bestrahlung mit fokussiertem Ultraschall und Ultraschall-Bildgebung (US 4 955 365),

- nichtinvasive Bestrahlung mit Ultraschall mittels eines nach dem "cross fire"-Prinzip aufgebauten Applikators und Temperaturmessung im Zielgebiet (DE 31 50 513)

- nichtinvasive Bestrahlung mit fokussiertem Ultraschall unter US-Bildgebung (EP 0 170 416), und

- Laser-Behandlung über Urethra-Sonde (WO 90.13333).

Bei Thermotherapie soll eine Übererwärmung eines größeren Volumens der Prostata mit nachfolgender Nekrotisierung bewirkt werden. Die Behandlung erfolgt über einen Zeitraum von Minuten bis etwa eine Stunde. Durch die Bestrahlung wird das Gewebe im zu behandelnden Bereich auf Temperaturen über 45° C bis etwa 60° C erwärmt und gehalten, so daß eine Schädigung mit dem Ziel einer Reduzierung des vermehrten Gewebes bewirkt wird. Im Falle der Hyperthermie wird dagegen eine Temperatur von 45° C nicht oder jedenfalls nicht wesentlich überschritten.

Wegen der üblicherweise radialen symmetrischen Abstrahlung von Mikrowellen-Antennen mit nach außen langsam abfallender Wirkung ist eine relativ scharfe Begrenzung der Wirkzone und eine Aussparung zu schonender Bereiche erwünscht. Neben der Kühlung der Urethra wird z.B. die Verwendung strahlenreflektierender Flüssigkeiten vorgeschlagen (EP 0 370 890). Eine fortlaufende Überwachung der resultierenden Erwärmung erfolgt mit in Rektum und Urethra eingeführten Temperatur- Sonden. Bei auf Mikrowellenbasis arbeitenden Therapieeinrichtungen sind aufwendige faseroptische Thermometer erforderlich.

Die Begrenzung der Wirkung auf die Prostata ist bei Hyperthermie mit transrektalen Applikatoren unbedriedigend. Thermische Schädigung von Gewebe außerhalb der Prostata kann auftreten.

Die Wirkung von fokussiertem Ultraschall ist wie die der Mikrowelle ebenfalls überwiegend thermisch und führt zu Nekrosen mit einer Ausdehnung von etwa 2 mm Durchmesser und 20 mm Länge. Für eine Volumenbehandlung müssen mehrere Nekrosen nebeneinander erzeugt werden. Die Zielbestimmung mittels Ultraschall-Bild liefert mit der gebräuchlichen Technik nur ein zweidimensionales Schnittbild. Die dreidimensionale Darstellung erfordert einen hohen Geräte- und Zeitaufwand und ist noch nicht klinisch verfügbar. Eine sofortige Feststellung der Lage des therapeutischen Wirkungsbereiches des Ultraschalls ist nur nach Eintritt einer Schädigung möglich.

Der Erfindung, wie sie im Anspruch 1 definiert wird, liegt die Aufgabe zugrunde, eine Therapieeinrichtung der eingangs genannten Art zur Therapie von Gewebe, insbesondere von Prostata-Gewebe bei BPH-Erkrankung, so auszubilden, daß es auf einfache und möglichst exakte Weise möglich ist, den Körper des Patienten und den therapeutischen Wirkbereich des Ultraschalls relativ zueinander so auszurichten, daß sich zumindest ein Teil des zu behandelnden Gewebes im therapeutischen Wirkbereich des Ultraschalls befindet. Außerdem soll nach Möglichkeit eine kontinuierliche Überwachung der Intensität des Ultraschalls erfolgen. Weiter soll die Behandlung möglichst wenig invasiv erfolgen.

Lösungen der Aufgabe sind in den Patentansprüchen angegeben.

Innerhalb des zu behandelnden Gewebes, speziell der Prostata bzw. eines Prostata-Lappens, wird ein begrenztes Volumen durch Ultraschall-Einwirkung gezielt behandelt, d.h. geschädigt, wobei

- empfindliche Strukturen, wie im Fall der Prostata die Urethra, durch Kühlung geschützt werden und/oder

- vor und eventuell während der therapeutischen Beschallung durch mindestens einen in das Gewebe, im Fall der Prostata bevorzugt in die Urethra, eingeführten miniaturisierten akustischen Drucksensor (Hydrophon) eine Messung und/oder Überwachung des Ultraschalls erfolgt und nach Vergleich mit den an diesem Ort erwünschten Werten gegebenenfalls Ort und/oder Orientierung und/oder Ansteuerung der Quelle des therapeutischen Ultraschalls verändert werden.

Die Behandlung erfolgt über einen Zeitraum von Minuten bis etwa eine Stunde. Durch die Beschallung wird das Gewebe im Zielvolumen auf Temperaturen über 45° C bis etwa 60° C erwärmt und gehalten, so daß eine Schädigung mit dem Ziel einer Reduzierung des vermehrten Gewebes bewirkt wird. Vor Beginn der eigentlichen Behandlung wird die Einstrahlung jedes Wandlers für sich und eventuell die aller Wandler mit dem (den) akustischen Sensor(en) gemessen und die Anordnung der Wandler gegebenenfalls korrigiert. Dabei kann in noch zu beschreibender Weise anstelle des bei der Therapie abgestrahlten Ultraschalls ein Pilotsignal verminderter Intensität abgestrahlt werden. Die Wandler können abdominal (auf der Bauchdecke) und/oder im Rektum und/oder perineal (Bereich zwischen Skrotum und After) appliziert sein. Die Lage des Zielgebietes bzw. die Anordnung der Wandler kann zusätzlich durch- Ultraschall-Bildgebung überprüft werden, wobei die entsprechenden Diagnostik-Applikatoren ebenfalls in den vorgenannten Bereichen appliziert werden. Während der Behandlung können die akustischen Werte und die Temperatur an verschiedenen Punkten (Urethra, Rektum) überwacht werden. Das kann bei therapiegemäßer Beschallung (alle Wandler in Betrieb) erfolgen oder aber auch durch kurzzeitigen Betrieb nur eines Wandlers (kurzzeitige Unterbrechung der Therapie).

Miniaturisierte akustische Sensoren und Temperaturfühler können in einem Katheter zusammengefaßt werden. Bei Einführung in die Urethra kann in ähnlicher Weise wie dies bei bekannten auf Mikrowellenbasis arbeiten Geräten damit die Kühlung verbunden werden. Die Kühlflüssigkeit gewährleistet eine gute akustische Einkopplung in den Katheter. Miniaturisierte akustische Sensoren oder Sensorarrays können basierend auf polymeren Piezofolien (z.B. Polyvinylidenflourid, kurz PVDF) oder Piezokeramik (z.B. Blei-Zirkonat-Titanat, kurz PZT) aufgebaut werden. Wegen des Fehlens von Mikrowellen-Strahlung kann die Temperaturmessung preiswert mit Thermoelementen, NTCs oder dergleichen erfolgen.

Die Begrenzung des wirksam beschallten Volumens kann entweder mit einem Ultraschallwandler geeigneter Fokussierung erreicht werden, oder durch Überlagerung der Schallfelder mehrerer Wandler. Die Fokuszone (-3dB Zone bezüglich Leistungsmaximum) eines fokussierenden Wandlers hat für harmonische Wellen einen

$$\text{radialen Durchmesser } d_{-3dB} = 1{,}22 \; c/f \times (F/D) \text{ und eine axiale Länge } Z_{-3dB} = 7 \times (F/D)^2$$

mit Schallgeschwindigkeit c (in Gewebe etwa 1500 m/s), Frequenz f, Durchmesser D der Quelle und Brennweite F. Für einen Wandler mit D = 12 cm, F = 8 cm und f = 500 kHz ergibt sich $d_{-3dB}$ = 2,5 mm und $z_{-3dB}$ = 9 mm. Strahlt der Wandler mit einer Leistung von 0,1 W/cm$^2$ ab, so ergibt sich unter Zugrundelegung eines geschätzten Wirkungsgrades von 25% als Folge von Absorption im vorgelagerten Gewebe (Fett, Muskel) und extrafokaler Strahlung im Fokus eine Leistung von etwa 60 W/cm$^2$. Davon werden bei Vernachlässigung nichtlinearer Effekte etwa 25% in der Fokuszone unter Wärmeentwicklung absorbiert. Eine therapeutische Wirkung wird im Minutenbereich erzielt. Die wirksame Fokuszone ist für diesen Fall ein Einzelwandler von länglicher Form.

Durch Überlagerung mehrerer Schallfelder aus unterschiedlichen Richtungen kann eine kompaktere Fokus-Geometrie erreicht werden. So kann mit drei Wandlern bei Beschallung aus etwa orthogonalen Richtungen die Fokuszone angenähert kugelförmig werden. Möglichkeiten zur Beschallung der Prostata sind aus den Richtungen Perineum, Unterbauch (in Rückenlage, von seitlich/oben durch die Blase, unter Umgehung des Schambeinknochens) und Rektum gegeben. Diese Richtungen sind etwa orthogonal. Dabei ist ein wesentlicher Vorteil dieser Anordnung, daß die Apertur des Einzelwandlers relativ klein sein kann und störenden Strukturen (Knochen, Gasvolumen) im Schallweg ausgewichen werden kann. Wird nur ein stark fokussierender Wandler mit notwendiger größerer Apertur verwandt, so ist eine Beschallung dieser Strukturen nur schwer zu vermeiden. Wird als Einzelwandler ein unfokussierter ebenen Schwinger verwendet, so hat sein natürlicher Fokus eine Breite

$$d_{-3dB} = D/3 \text{ in einem Abstand } F = 0{,}75 \; D^2 \times f/4c.$$

Für einen Wandler mit f = 875 kHz und D = 2,5 cm ergibt sich $d_{-3dB}$ = 8 mm und F = 7 cm.

Bei Abstrahlung einer Leistung von etwa 3 W/cm$^2$ wird im Gewebe eine merkliche Erwärmung, jedoch ohne Schädigung, spürbar. Werden die Felder von drei dieser Wandler überlagert, so bildet sich eine kugelförmige Fokuszone mit etwa 8 mm Durchmesser. Die in Form von Wärme in diesem Volumen absorbierte Leistung beträgt etwa 7 W. Eine therapeutische Wirkung ist nach mehreren Minuten zu erwarten.

Abhängig vom Applikationsort und Schallweg zum Ziel können die Wandler unterschiedliche Apertur, Fokussierung und Frequenz besitzen sowie unterschiedlich angesteuert werden. So hat die Kombination eines schwach fokussierenden Wandlers (abdominale oder perineale Applikation) mit einem stark fokussierten Wandler (rektale Applikation) zur Folge, daß ein Volumenbereich auf eine Temperatur knapp unter 45° C (vor)gewärmt werden kann und nur ausgewählte Zielpunkte durch das überlagerte Fokusfeld nekrotisiert werden. Vorteil ist, daß der rektale Applikator, der sich nahe an der Prostata befindet, eine geringere Leistung besitzen muß als bei ausschließlicher Beschallung vom Rektum aus. Zum Beipiel kann ein Wandler mit 875 kHz mit einem 2 MHz-Wandler kombiniert werden. Mit Durchmesser und Brennweite von je 40 mm ergibt sich eine -3dB-Zone von etwa 1 mm Durchmesser und 5 mm Länge.

Die Orientierung der einzelnen Wandler relativ zur Prostata erfolgt so, daß die Wandler zunächst grob in Zielrichtung an der Körperoberfläche appliziert werden. Sie sind jeweils um zwei Ebenen schwenkbar in einem Gehäuse mit flüssiger Vorlaufstrecke und Koppelmembran untergebracht. Rektal applizierte Wandler sind bevorzugt in einer Richtung schwenkbar und um die Längsachse der Sonde drehbar angeordnet.

Unter Aussenden eines Pilotsignals werden die Wandler geschwenkt bzw. gedreht, bis Signale von dem (den) in der Urethra liegenden Hydrophon(en) empfangen werden. Durch Feineinstellung der Richtung werden die Signale optimiert. Abhängig davon, welcher Bereich der Prostata beschallt werden soll, werden maximales Druck- oder Leistungssignal, linke oder rechte -3dB Flanke davon oder dergleichen gewählt. Mit mehreren längs des Urethers verteilten Hydrophonen ergibt sich eine höhere Genauigkeit und Eindeutigkeit.

Ausführungsbeispiele der Erfindung sind in den beigefügten Zeichnungen am Beispiel einer Therapieeinrichtung zur Behandlung der benignen Prostatahyperplasie dargestellt. Eszeigen:

Fig. 1    in grob schematischer Darstellung im Längsschnitt den Körper eines Patienten und eine an diesen applizierte erfindungsgemäße Therapieeinrichtung,

Fig. 2    einen der Ultraschallwandler der Therapieeinrichtung gemäß Fig. 1 in vergrößerter schematischer Darstellung im Längsschnitt,

Fig. 3    ein Detail der Therapieeinrichtung gemäß Fig. 1 in stark vergrößerter schematischer Darstellung im Längsschnitt, und

Fig. 4 und 5    in zur Fig. 1 analoger Darstellung zwei weitere erfindungsgemäße Therapieeinrichtungen.

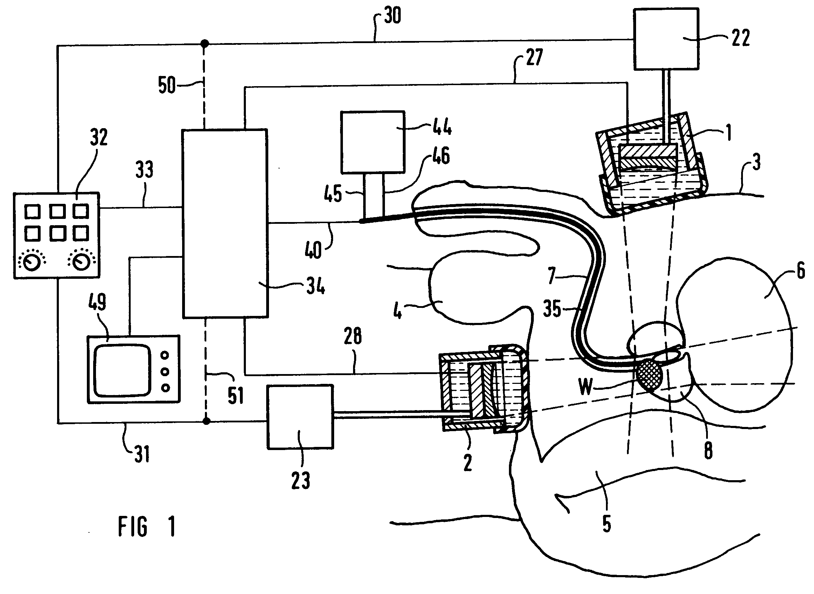

Die Einrichtung gemäß Fig. 1 weist zwei therapeutische Ultraschallwandler 1 und 2 auf, die an den in Fig. 1 im Querschnitt angedeuteten Körper 3 eines Patienten appliziert sind. Die Applikation des Ultraschallwandlers 1 erfolgt abdominal im Bereich des Unterbauchs des Patienten, der übrigens in nicht näher dargestellter Weise liegend, beispielsweise auf einem urologischen Behandlungstisch, gelagert ist. Der Ultraschallwandler 2 ist perineal, d.h. zwischen dem Skrotum 4 und dem Rektum 5 appliziert. Wie anhand der in Fig. 1 strichliert eingetragenen "Randstrahlen" des von den Ultraschallwandlern 1 und 2 ausgehenden Ultraschalls ersichtlich ist, sind die Ultraschallwandler 1 und 2 jeweils auf die der Harnblase 6 im Bereich der Mündung der Urethra 7 vorgelagerte und die Urethra 7 umgebende Prostata 8 ausgerichtet. Die Prostata 8 weist eine benigne Hyperplasie (gutartige Vergrößerung) auf.

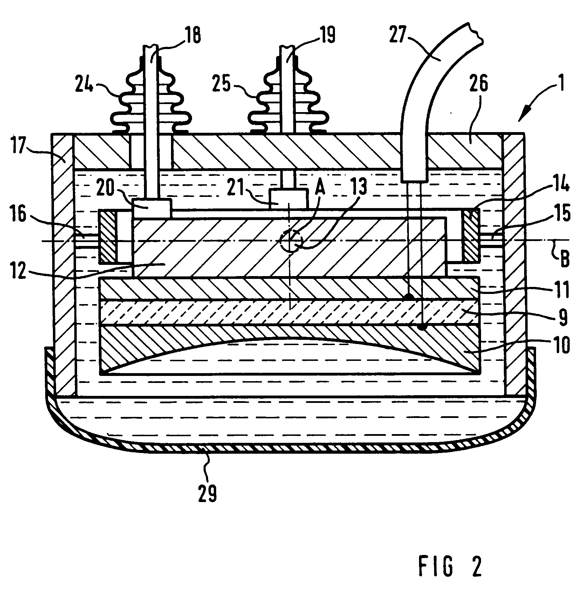

Die Ultraschallwandler 1 und 2 sind so, wie dies in Fig. 2 am Beispiel des Ultraschallwandlers 1 dargestellt ist, aufgebaut. Demnach ist als Ultraschallerzeuger eine scheibenförmige Piezokeramik 9 vorgesehen. Diese ist an ihrer Vorderseite in an sich bekannter Weise mit einer plan-konkaven akustischen Sammellinse 10 versehen, die der Fokussierung des erzeugten Ultraschalls dient. An ihrer Rückseite ist die Piezokeramik 9 in an sich bekannter Weise mit einem Backing 11 gekoppelt. Das Backing 11 ist seinerseits mit einem kreisscheibenförmigen Tragkörper 12 verbunden, der mittels zweier Zapfen, einer davon ist in Fig. 2 strichliert angedeutet und mit dem Bezugszeichen 13 versehen, um eine rechtwinklig zur Zeichenebene stehende Achse A schwenkbar in einem Zwischenring 14 gelagert. Der Zwischenring 14 ist seinerseits mittels zweier Zapfen 15 und 16 um eine rechtwinklig zur Achse A verlaufende Achse B schwenkbar in einem rohrförmigen Gehäuse 17 gelagert. Im Falle des Ultraschallwandlers 1 gemäß Fig. 2 schneiden sich die Achsen A und B.

Mittels zweier Stangen 18 und 19, die mit Kugelgelenken 20 bzw. 21 an dem Tragkörper 12 bzw. dem Zwischenring 14 angelenkt sind, besteht die Möglichkeit, die Hauptschallausbreitungsrichtung des erzeugten Ultraschalls relativ zu dem Gehäuse 17 durch Schwenken um die Achse A und/oder die Achse B zu variieren. Die Stangen 18 und 19, die Bestandteile in der Fig. 1 schematisch angedeuteter Verstelleinheiten 22 und 23 sind, sind mit Hilfe von Faltenbälgen 24 bzw. 25 flüssigkeitsdicht durch einen das Gehäuse 17 flüssigkeitsdicht abschließenden Boden 26 nach außen geführt.

In nicht näher dargestellter Weise ebenfalls flüssigkeitsdicht ist ein Versorgungskabel 27 durch den Boden 26 geführt, über das die Piezokeramik 9 in der zur Erzeugung von Ultraschall erforderlichen Weise mit einer Ansteuerspannung versorgt wird. Das Versorgungskabel 27 ist zweiadrig ausgeführt, wobei eine Ader mit einer an der Vorderseite und die andere Ader mit einer an der Rückseite der Piezokeramik 9 vorgesehenen, in Fig. 2 nicht dargestellten Elektrode verbunden ist. Das Versorgungskabel 27 und das entsprechende, zu dem Ultraschallwandler 2 gehörige Versorgungskabel 28 sind in Fig. 1 nur schematisch dargestellt.

An seinem dem Boden 26 abgewandten Ende ist das Gehäuse 17 mittels einer flexiblen Ankoppelmembran 29 flüssigkeitsdicht verschlossen. Diese dient dazu, den Ultraschallwandler 1 zur akustischen Ankoppelung an die Körperoberfläche des Patienten anzupressen. Der gesamte Innenraum des Gehäuses 17 ist mit einem flüssigen akustischen Ausbreitungsmedium für den erzeugten Ultraschall ausgefüllt. Sowohl das akustische Ausbreitungsmedium als auch das Material der Ankoppelmembran 29 weisen vorzugsweise eine akustische Impedanz auf, die der des Körpergewebes des Patienten im wesentlichen entspricht. Geeignete Materialien sind beispielsweise Wasser und EPDM-Gummi.

Die Verstelleinheiten 22 und 23 gestatten neben den Schwenkbewegungen um die Achsen A und B, die motorisch ausgeführt werden, unter Deformation der Ankoppelmembran 29 eine Verschiebung der Ultraschallwandler 1 und 2 in Richtung ihrer jeweiligen Mittelachse. Die zur Realisierung der beschriebenen motorischen Verstellbewegungen erforderlichen Maßnahmen vermag der Fachmann aufgrund seines Fachwissens zu treffen. Nähere Einzelheiten sind daher nicht beschrieben. Die Ultraschallwandler 1 und 2 sind in nicht näher dargestellter Weise an beispielsweise stativartig ausgebildeten Tragvorrichtungen angebracht, die abgesehen von den mittels der Verstelleinrichtungen 22 und 23 bewirkbaren motorischen Verstellbewegungen eine manuelle Ausrichtung der Ultraschallwandle 1 und 2 relativ zum Körper 3 des Patienten gestatten.

Die Verstelleinheiten 22 und 23 stehen über Steuerleitungen 30, 31 mit einer Bedieneinheit 32 in Verbindung. Diese ist unter anderem mit den zur Ausführung der beschriebenen Verstellbewegungen der Verstelleinheiten 22 und 23 erforderlichen Bedienelementen versehen. Die Bedieneinheit 32 weist außerdem Bedienelemente auf, mittels derer es möglich ist, die Intensität des mittels der Ultraschallwandler 1 und 2 erzeugten Ultraschalls unabhängig voneinander einzustellen. Der jeweils gewählten Einstellung entsprechende Informationen gelangen über eine Leitung 33 zu einer Steuereinheit 34, die unter anderem die elektrischen Generatoreinrichtungen enthält, die die Wechselspannungen erzeugen, die erforderlich sind, um die Ultraschallwandler 1 und 2 zur Erzeugung von Ultraschall ansteuern zu können, und die über die Versorgungskabel 27 und 28 zu den Ultraschallwandlern 1 und 2 gelangen.

Die Ultraschallwandler 1 und 2 werden mit Hilfe der Verstelleinheiten 22 und 23 derart ausgerichtet, daß die Hauptausbreitungsrichtungen des von den Ultraschallwandlern 1 und 2 ausgehenden Ultraschalls derart ausgerichtet sind, daß sich der von den Ultraschallwandlern 1 und 2 ausgehende Ultraschall im Bereich der Prostata 8 überlagert. Der von den Ultraschallwandlern 1 und 2 ausgehende Ultraschall ist jeweils nur schwach fokussiert. Die Ultraschallwandler 1 und 2 weisen also einen nur geringen Fokussierungsgrad auf, der im Falle beider Ultraschallwandler 1 und 2 wenigstens im wesentlichen gleich ist. Die Fokuszonen des von den Ultraschallwandlern 1 und 2 ausgehenden Ultraschalls, die sich im Bereich der durch die jeweiligen "Randstrahlen" gebildeten Einschnürung befinden, überlappen einander zumindest teilweise. Sie weisen etwa die gleichen Abmessungen und etwa die gleiche Gestalt auf. Die Intensität sowohl des mittels des Ultraschallwandlers 1 als auch des mittels des Ultraschallwandlers 2 erzeugten Ultraschalls ist so eingestellt, daß nur innerhalb desjenigen Bereiches, in dem sich der mittels der Ultraschallwandler 1 und 2 erzeugte Ultraschall überlagert, Ultraschallintensitäten erreicht werden können, die zur Erzielung eines therapeutischen Effektes ausreichen.

In Abhängigkeit davon, ob die Behandlung als Hyperthermie oder als Thermotherapie erfolgt, werden die Ultraschallintensitäten derart gewählt, daß eine Erwärmung des Prostatagewebes auf maximal 45° C oder höhere Temperaturen, vorzugsweise 45° C bis etwa 60° C, erfolgt.

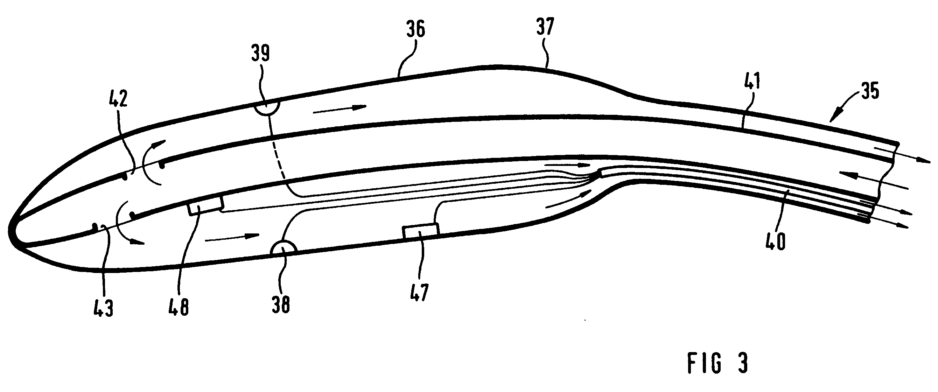

Unter anderem um die im Prostatagewebe auftretenden Temperaturen überwachen zu können, weist die Therapieeinrichtung einen Katheter 35 auf, der in die Urethra 7 des Patienten so weit eingeführt wird, daß sich sein distales Ende in demjenigen Bereich der Urethra 7 befindet, der von der Prostata 8 umgeben ist. Das distale Ende 36 des Katheters 35 ist in Fig. 3 im Längsschnitt dargestellt. An der Innenseite seiner schlauchartigen Außenwand 37 sind zwei Temperatursensoren 38 und 39, beispielsweise NTC-Widerstände, appliziert, die der Temperaturmessung dienen. Die der gemessenen Temperatur entsprechenden elektrischen Signale gelangen über eine schematisch angedeutete Meßleitung 40 zur Steuereinheit 34. Die mehradrige Meßleitung 40 ist in Fig. 1 als einadrige Leitung schematisiert. Der Katheter 35 ist doppelläufig ausgeführt, d.h. innerhalb der Außenwand 37 ist eine schlauchförmige Innenwand 41 koaxial angeordnet. Innerhalb der Innenwand 41 wird ein Kühlmedium, beispielsweise Wasser, zu dem distalen Ende 36 des Katheters 35 gefördert und tritt dort durch Überströmöffnungen 42 und 43 in den zwischen der Innenwand 41 und der Außenwand 37 befindlichen Raum über und strömt vom distalen Ende 36 zum proximalen Ende des Katheters 35 zurück. Die Kühlmittelströmung ist in Fig. 3 durch Pfeile angedeutet. Das Kühlmittel dient dazu, die Urethra 7 vor einer thermischen Schädigung zu bewahren. Das Kühlmittel strömt in einem geschlossenen Kreislauf durch ein in Fig. 1 schematisch angedeutetes Kühlaggregat 44, das über zwei Leitungen 45 und 46 mit dem durch die Innenwand 37 begrenzten Kanal bzw. dem durch die Innenwand 41 und die Außenwand 37 begrenzten Kanal verbunden ist. Die Meßleitung 40 verläuft übrigens in dem zwischen der Innenwand 41 und der Außenwand 37 begrenzten Kanal.

Im Bereich des distalen Endes 36 des Katheters 35 sind außerdem zwei akustische Drucksensoren 47 und 48, vorzugsweise in Form von miniaturisierten Hydrophonen, angeordnet.

Der Drucksensor 47 ist an die Innenseite der Außenwand 37 angebracht. Der Drucksensor 48 ist auf die Außenseite der Innenwand 41 appliziert. Andere Anordnungen der Drucksensoren 47, 48 sind möglich. Die Drucksensoren 47, 48, in die eventuell erforderliche Vorverstärker integriert sein können, sind vorzugsweise unter Verwendung von piezoelektrisch aktivierter Polymerfolie, beispielsweise Polyvinylidenfluorid (PVDF), aufgebaut.

Die Drucksensoren 47, 48, die über das in dem Katheter 35 strömende Medium akustisch gut mit dem umgebenden Gewebe gekoppelt sind, dienen dazu, die Ultraschallwandler 1 und 2 in bezug auf den jeweils zu behandelnden Bereich der Prostata 8 optimal auszurichten. Dies geschieht in der Weise, daß zunächst nur der Ultraschallwandler 1 zur Abgabe von Ultraschall angesteuert wird und mittels der Verstelleinheit 22 so ausgerichtet wird, daß wenigstens einer der Drucksensoren 47, 48 ein maximales Ausgangssignal liefert. Die Ausgangssignale der Drucksensoren 47, 48 gelangen übrigens ebenfalls über die Meßleitung 40 zu der Steuereinheit 34, die mit einem Monitor 49 verbunden ist, der unter anderem der Anzeige der mittels der Drucksensoren 47, 48 gemessenen Drücke, die der Intensität des Ultraschalls proportional sind, dient. Anschließend wird anstelle des Ultraschallwandlers 1 allein der Ultraschallwandler 2 zur Abgabe von Ultraschall aktiviert und mittels der Verstelleinrichtung 23 ebenfalls derart ausgerichtet, daß wenigstens einer der Drucksensoren 47, 48, vorzugsweise der gleiche wie im Falle des Ultraschallwandlers 1, ein maximales Ausgangssignal liefert. Es kann nun davon ausgegangen werden, daß sich bei Aktivierung beider Ultraschallwandler 1 und 2 der Bereich maximaler Intensität des Ultraschalls im Bereich des distalen Endes 36 des Katheters 35, insbesondere im Bereich des das maximale Ausgangssignal liefernden Drucksensors 47 bzw. 48, befindet. Um mit Sicherheit eine Schädigung von gesundem Gewebe zu vermeiden, kann vorgesehen sein, daß die Ultraschallwandler 1 und 2 während des beschriebenen Ausricht-Vorganges lediglich ein sogenanntes Pilotsignal aussenden, bei dem es sich um Ultraschall so weit verminderter Intentität handelt, daß mit Sicherheit eine Gewebebeschädigung ausgeschlossen werden kann.

Zur Durchführung der Therapie werden beide Ultraschallwandlerr 1 und 2 derart angesteuert, daß sich zumindest im Bereich der maximalen Intensität des Ultraschalls eine Ultraschallintensität ergibt, die ausreicht, um das Gewebe auf eine Temperatur zu erwärmen, die zur Erzielung eines therapeutischen Effektes erforderlich ist. Die Größe des therapeutischen Wirkbereiches, also desjenigen Bereiches, in dem die Ultraschallintensität ausreicht, um das zu behandelnde Gewebe auf die zur Erzielung eines therapeutischen Effekts erforderliche Temperatur zu erwärmen, läßt sich durch Verändern der Ultraschallintensität eines oder beider Ultraschallwandler 1, 2 verändern, wobei eine Erhöhung der Ultraschallintensität eine Vergrößerung des therapeutischen Wirkbereiches nach sich zieht. In Fig. 1 ist der therapeutische Wirkbereich W beispielhaft durch Kreuzschraffur angedeutet.

Durch geeignete Wahl der Ultraschallintensitäten des mittels der Ultraschallwandler 1 und 2 erzeugten Ultraschalls läßt sich eine dem jeweiligen Behandlungsfall entsprechende Größe des therapeutischen Wirkbereiches W einstellen. Außerdem läßt sich der therapeutische Wirkbereich W durch geringfügige Verstellung der Ultraschallwandler 1 und 2 mittels der Verstellmittel 22 und 23 relativ zueinander in seiner Lage verändern, so daß für den Fall, daß der therapeutische Wirkbereich W kleiner als die Prostata 8 ist, der therapeutische Wirkbereich W nach und nach so verlagert werden kann, daß die gesamte Prostata 8 behandelt wird.

Aufschluß über die jeweils vorliegende Größe des therapeutischen Wirkbereiches W geben die auf dem Monitor 49 angezeigten, mittels der Drucksensoren 47, 48 gemessenen Druckwerte. Zusätzlich erfolgt eine Überwachung des Therapievorganges anhand der mittels der Temperatursensoren 38, 39 gemessenen Temperaturwerte, die ebenfalls auf dem Monitor 49 angezeigt werden. Auch die gemessenen Temperaturwerte werden auf dem Monitor 49 angezeigt.

Die gemessenen Druckwerte können auch herangezogen werden, um Aufschluß über die momentane Lage des therapeutischen Wirkbereiches W zu erhalten, und zwar, indem zunächst die für die eingestellten Ultraschallintensitäten maximal möglichen Druckwerte in der zuvor beschriebenen Weise bestimmt werden und die sich bei einer Verstellung der Ultraschallwandler 1 und 2 mittels der Verstellmittel 22 und 23 ergebenden Druckwerte mit den Maximalwerten verglichen werden. Aus der Höhe der Abweichung der aktuellen Meßwerte von den Maximalwerten läßt sich erkennen, wenn der therapeutische Wirkbereich in eine nicht mehr behandlungsbedürftige Gewebezone verlagert wurde.

In diesem Zusammenhang kann vorgesehen sein, daß die Steuereinheit 34 die gemessenen Druckwerte mit einem Schwellwert vergleicht, der in Abhängigkeit von den mittels der Bedieneinheit 32 eingestellten Ultraschallintensitäten bestimmt wird, und dessen Unterschreitung darauf hindeutet, daß der therapeutische Wirkbereich W in den Bereich gesunden Gewebes verlagert wurde. Der Schwellwert kann von dem Bedienpersonal unter Berücksichtigung der Größe der zu behandelnden Prostata 8 mit Hilfe von Tabellen oder dergleichen bestimmt und über die Bedieneinheit 32 eingegeben werden. Es kann aber auch die Steuereinheit 34 derart ausgebildet sein, daß sie den Schwellwert selbsttätig, gegebenenfalls nach Eingabe der Größe der zu behandelnden Prostata 8, errechnet.

Zweckmäßigerweise ist die Steuereinheit 34 derart ausgebildet, daß sie bei Unterschreitung des Schwellwertes die Ansteuerung der Ultraschallwandler 1 und 2 blockiert und eine erneute Ansteuerung der Ultraschallwandler zur Erzeugung von Ultraschall mit einer solchen Ultraschallintensität, daß ein therapeutischer Effekt erzielbar ist, erst nach einer erneuten Ausrichtung der Ultraschallwandler 1 und 2 in der oben beschriebenen Weise ermöglicht.

Neben dem Vergleich der mittels der Drucksensoren 47, 48 gemessenen Druckwerte mit einem Schwellwert nimmt die Steuereinheit 34 auch einen Vergleich der mittels der Temperatursensoren 38, 39 gemessenen Temperaturen mit einem oberen und einem unteren Schwellwert vor. Die Schwellwerte für die Temperatur können ebenfalls über die Bedieneinheit 32 eingegeben werden. Der obere Schwellwert entspricht der dem jeweiligen Behandlungsfall angepaßten Maximaltemperatur, der untere Schwellwert der dem jeweiligen Behandlungsfall entsprechend gewählten Mindesttemperatur, auf die das zu behandelnde Gewebe wenigstens aufgeheizt werden soll. Wird der obere Schwellwert der Temperatur überschritten, wird die Ansteuerung der Ultraschallwandler 1 und 2 blockiert und erst wieder freigegeben, wenn die Temperatur um ein bestimmtes Maß, beispielsweise 5° C, unter den oberen Schwellwert gesunken ist. Wird der untere Schwellwert unterschritten, wird ein entsprechender Hinweis auf dem Monitor 49 ausgegeben, der das Bedienpersonal darauf hinweist, daß ein therapeutischer Effekt unter Umständen nicht mehr erzielt wird.

Die Steuereinheit 34 kann auch derart ausgebildet sein, daß die Verstellung der Ultraschallwandler 1 und 2 mittels der Verstelleinheiten 22 und 23 nicht durch entsprechende Betätigung der Bedieneinheit 32, sondern durch die Steuereinheit 34 unter Auswertung der Ausgangssignale der Drucksensoren 47, 48 erfolgt. Dies ist in der Fig. 1 dadurch angedeutet, daß die Steuerleitungen 30 und 31 über strichliert angedeutete Leitungen 50, 51 mit der Steuereinheit 34 verbunden sind.

Die im folgenden beschriebenen Ausführungsbeispiele stimmen mit dem zuvor beschriebenen in wesentlichen Punkten überein, weshalb gleiche oder ähnliche Elemente die gleichen Bezugszeichen tragen.

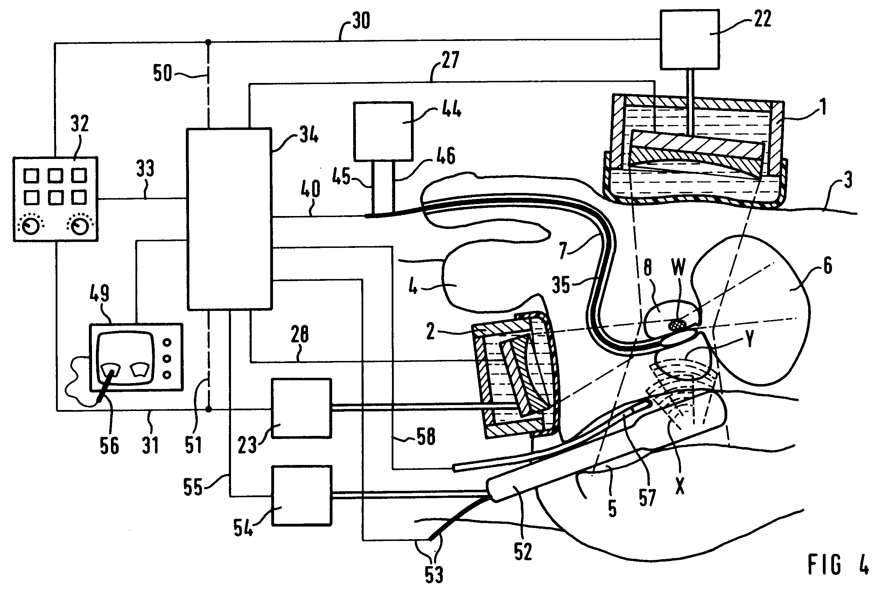

Während im Falle des zuvor beschriebenen Ausführungsbeispieles die Ultraschallwandler 1 und 2 nur schwach fokussierten Ultraschall aussenden und die Fokuszonen im Falle beider Ultraschallwandler 1 und 2 im wesentlichen die gleichen Abmessungen aufweisen, weisen die Ultraschallwandler 1 und 2 im Falle der Fig. 4, wie die strichliert eingetragenen "Randstrahlen" des von den Ultraschallwandlern 1 und 2 ausgehenden Ultraschalls zeigen, einen unterschiedlichen Fokussierungsgrad auf. Der Ultraschallwandler 1 weist einen geringeren Fokussierungsgrad als der Ultraschallwandler 2 auf und sendet schwach fokussierten Ultraschall mit einer vergleichsweise großen Fokuszone aus. Der von dem Ultraschallwandler 2 ausgehende Ultraschall ist dagegen stark fokussiert und weist eine Fokuszone auf, die wesentlich kleiner als die des von dem Ultraschallwandler 2 ausgehenden Ultraschalls ist. Während der Therapie sind die Ultraschallwandler 1 und 2 relativ zueinander derart ausgerichtet, daß die zu dem Ultraschallwandler 2 gehörige Fokuszone innerhalb der zu dem Ultraschallwandler 1 gehörigen Fokuszone liegt. Nachdem die Ultraschallwandler 1 und 2 anhand der Ausgangssignale der Drucksensoren 47 und 48 vor Beginn der Therapie in der im Zusammenhang mit dem zuvor beschriebenen Ausführungsbeispiel erläuterten Weise mittels der Verstelleinheiten 22 und 23 ausgerichtet sind, erfolgt im Falle des Ausführungsbeispieles gemäß Fig. 4 die Verlagerung der therapeutischen Wirkzone W, die im Vergleich zum zuvor beschriebenen Ausführungsbeispiel sehr klein ist, dadurch, daß unter Beibehaltung der Ausrichtung des Ultraschallwandlers 1 relativ zu dem Körper 3 die Ausrichtung des Ultraschallwandlers 2 mittels der Verstelleinheit 23 derart verändert wird, daß die therapeutische Wirkzone W nach und nach die zu behandelnden Bereiche des Gewebes der Prostata 8 abtastet. Dabei bleibt die kleinere Fokuszone des von dem Ultraschallwandler 2 ausgehenden Ultraschalls stets innerhalb der größeren Fokuszone des von dem Ultraschallwandler 1 ausgehenden Ultraschalls; andernfalls wäre ein therapeutischer Effekt nicht gewährleistet.

Die Therapieeinrichtung gemäß Fig. 4 weist als zusätzliches Element eine für die rektale Applikation geeignete Ultraschall-Diagnostik-Sonde 52 auf, die in der in Fig. 4 dargestellten Weise in das Rektum 5 eingeführt wird. Mittels der Ultraschall-Diagnostik-Sonde 52 sind Ultraschall-Schnittbilder zweier einander schneidender, vorzugsweise kreissektorförmiger Körperschichten des Patienten erzeugbar. Die der Ultraschall-Diagnostik-Sonde 52 benachbarten Bereiche der kreissektorförmigen Körperschichten sind in Fig. 4 strichliert angedeutet und mit X bzw. Y bezeichnet. Die Ultraschall-Diagnostik-Sonde 52 wird im Rektum 5 derart plaziert, daß die in den Ultraschall-Schnittbildern dargestellten Körperschichten X, Y durch die Prostata 8 verlaufen. Die Ultraschall-Diagnostik-Sonde 52 enthält in nicht dargestellter Weise eine zur Erzeugung der beiden Ultraschall-Schnittbilder geeignete Ultraschall-Transducer-Anordnung, die über eine Steuer- und Signalleitung 53 mit der Steuereinheit 34 in Verbindung steht. Diese enthält die zur Ansteuerung der Ultraschall-Transducer-Anordnung und zur Verarbeitung der von dieser gelieferten Signale sowie zur Erzeugung der Ultraschall-Schnittbilder erforderliche Elektronik, die konventionell ausgebildet ist. Die beiden kreissektorförmigen Ultraschall-Schnittbilder werden in der in Fig. 4 angedeuteten Weise auf dem Monitor 49 dargestellt.

Die Ultraschall-Diagnostik-Sonde 52 ist übrigens an einem in Fig. 4 schematisch angedeuteten Positioniermechanismus 54 angebracht, der es gestattet, innerhalb eines bestimmten Verstellbereiches die Ultraschall-Diagnostik-Sonde 52 beliebig im Raum zu positionieren. Der Positioniermechanismus 54 verfügt über Positionsgeber, die über eine Leitung 55 der räumlichen Position der Ultraschall-Diagnostik-Sonde 52 entsprechende Signale an die Steuereinheit 34 geben. Beispielsweise kann der Positioniermechanismus in aus der Ultraschalltechnik (Compound-Scan) an sich bekannter Weise als Gelenkarm ausgebildet sein, dessen Gelenken jeweils Winkelgeber zugeordnet sind. Wenn also die Ultraschall-Diagnostik-Sonde 52 in das Rektum 5 des Patienten eingeführt und derart ausgerichtet wird, daß ein bestimmter zu behandelnder Bereich der Prostata 8 in den beiden Schnittbildern dargestellt wird, gehen der Steuereinheit 34 Signale zu, die die Lage der in den Schnittbildern dargestellten Körperschichten X und Y repräsentieren.

In einer mittels der Bedieneinheit 32 wählbaren ersten Betriebsweise wird in die auf dem Monitor 49 dargestellten Ultraschall-Schnittbilder jeweils eine die Lage des therapeutischen Wirkbereiches W repräsentierende Marke eingeblendet. Es besteht nun die Möglichkeit, durch entsprechende Betätigung der Bedieneinheit 32 die Ultraschallwandler 1 und 2 mittels der Verstelleinheiten 22 und 23 so auszurichten, daß der Wirkbereich W in einem bestimmten in den Ultraschall-Schnittbildern dargestellten Bereich des Gewebes der Prostata 8 liegt.

In einer zweiten, ebenfalls mittels der Bedieneinheit 32 anwählbaren Betriebsweise wird in die Ultraschall-Schnittbilder ebenfalls eine der Lage des therapeutischen Wirkbereiches W entsprechende Marke eingeblendet, die jedoch von der Ausrichtung der Ultraschallwandler 1 und 2 unabhängig ist. Die Ultraschall-Diagnostik-Sonde 52 wird dann so im Rektum 5 positioniert, daß sich das Abbild eines bestimmten zu behandelnden Gewebebereiches der Prostata 8 in den beiden Ultraschall-Schnittbildern jewils mit der der Position des Wirkbereiches W entsprechenden Markierung deckt. Ist dies der Fall, steuert auf eine entsprechende Betätigung der Bedieneinheit 32 hin die Steuereinheit 34 die Verstelleinheiten 22 und 23 derart an, daß die Ultraschallwandler 1 und 2 so ausgerichtet werden, daß sich der therapeutische Wirkbereich W des Ultraschalls in derjenigen Gewebezone der Prostata 8 befindet, deren Abbild sich in den Ultraschall-Schnittbildern mit der Markierung deckt.

In einer dritten mittels der Bedieneinheit 32 anwählbaren Betriebsweise besteht die Möglichkeit, mittels eines Lichtgriffels 56 in einem Ultraschall-Schnittbild oder beiden einen bestimmten Gewebebereich der Prostata 8 zu markieren, auf den auf eine entsprechende Betätigung der Bedieneinheit 32 hin die Steuereinheit 34 durch entsprechende Ansteuerung der Verstelleinheiten 22 und 23 den therapeutischen Wirkbereich W verlagert.

Im Falle der Therapieeinrichtung gemäß Fig. 4 ist übrigens zusätzlich zu der Ultraschall-Diagnostik-Sonde 52 eine Meßsonde 57 in das Rektum 5 des Patienten eingeführt, die in nicht dargestellter Weise wenigstens je einen Druck- und Temperatursensor enthält. Die mittels der Meßsonde 57 gewonnenen Meßwerte werden über eine Meßleitung 58 zu der Steuereinheit 34 übertragen. Die entsprechenden Druck- und Temperaturwerte werden zusätzlich zu den mittels des Katheters 35 gewonnenen Meßwerten auf dem Monitor 49 angezeigt. Auch bezüglich der mit der Meßsonde 57 gewonnenen Meßwerte kann in der im Zusammenhang mit den mittels des Katheters 35 gewonnenen Meßwerten beschriebenen Weise ein Vergleich mit Schwellwerten erfolgen, wobei eine Über- bzw. Unterschreitung der Schwellwerte die zuvor beschriebenen Folgen hat.

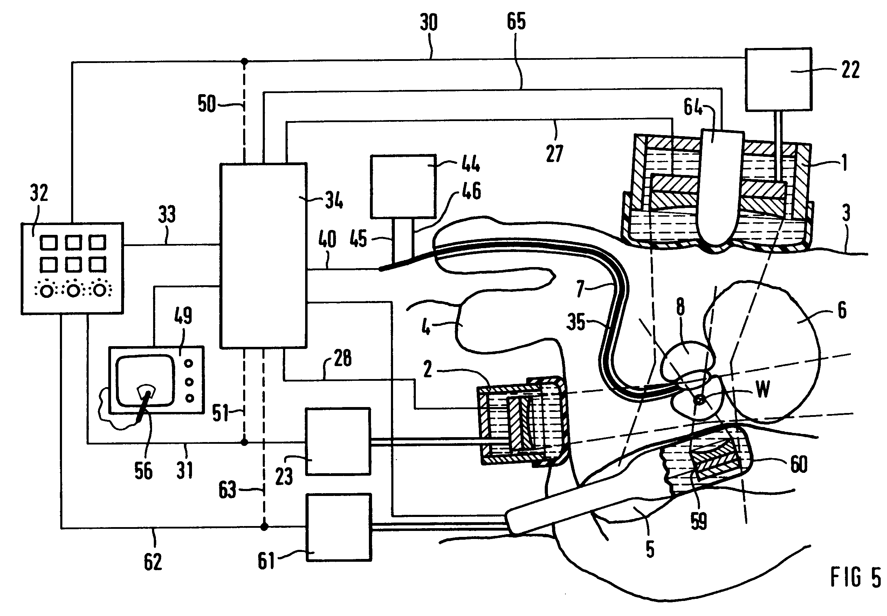

Die Therapieeinrichtung gemäß Fig. 5 unterscheidet sich von der gemäß Fig. 1 zunächst dadurch, daß ein zusätzlicher therapeutischer Ultraschallwandler 59 vorgesehen ist, der in einer für die rektale Applikation vorgesehenen Sonde 60 aufgenommen ist. Der Ultraschallwandler 59 ist in der in Fig. 5 teilweise aufgebrochenen Sonde 60 in nicht näher dargestellter Weise derart montiert, daß er um die Längsachse der Sonde 60 verdrehbar und um eine diese Längsachse vorzugsweise rechtwinklig schneidende Achse schwenkbar ist. Der Sonde 60 bzw. dem Ultraschallwandler 59 ist eine Verstelleinheit 61 zugeordnet, die über die Steuerleitung 62 von der Bedieneinheit 32 bzw. über die Leitung 63 von der Steuereinheit 34 angesteuert werden kann. Auch der Sonde 60 ist eine nicht dargestellte, beispielsweise stativartige Tragvorrichtung zugeordnet.

Bei den Ultraschallwandlern 1 und 2 handelt es sich wie im Falle der Therapieeinrichtung gemäß Fig. 1 um Wandler geringen Fokussierungsgrades, die also nur schwach fokussierten Ultraschall aussenden (siehe die in Fig. 5 strichliert eingetragenen "Randstrahlen" des von den Ultraschallwandlern 1 und 2 ausgehenden Ultraschalls). Im Gegensatz zu der Therapieeinrichtung gemäß Fig. 1 sind die Ultraschallintensitäten jedoch so gewählt, daß die Überlagerung des von den Ultraschallwandlern 1 und 2 ausgehenden Ultraschalls allein noch keinen therapeutischen Effekt nach sich zieht. Erst wenn zusätzlich eine Über-lagerung mit dem mittels des Ultraschallwandlers 59, der, wie die strichliert eingetragenen "Randstrahlen" des von ihm ausgehenden Ultraschalls zeigen, einen hohen Fokussierungsgrad aufweist, erzeugten stark fokussierten Ultraschalls erfolgt, ergibt sich ein therapeutischer Effekt, der in Anbetracht der starken Fokussierung des mittels des Ultraschallwandlers 60 erzeugten Ultraschalls auf einen sehr kleinen therapeutischen Wirkbereich W beschränkt ist. Die Ankoppelung des Ultraschallwandlers 1 erfolgt wieder am Unterbauch des Patienten, und zwar vorzugsweise in aus der Fig. 5 nicht ersichtlicher Weise von seitlich, so daß die Hauptausbreitungsrichtungen des von den Ultraschallwandlern 1, 2 und 59 ausgehenden Ultraschalls jeweils nahezu senkrecht zueinander stehen. Auf diese Weise ergibt sich ein therapeutischer Wirkbereich W von nahezu spärischer Gestalt.

Die Ausrichtung des therapeutischen Wirkbereiches W erfolgt anhand der Ausgangssignale des Katheters 35 in der im Zusammenhang mit der Ausführungsform gemäß Fig. 1 beschriebenen Weise; die Verlagerung des therapeutischen Wirkbereiches W in die zu behandelnden Bereiche des Gewebes der Prostata erfolgt vorzugsweise, indem die Ultraschallwandler 1 und 2 ihre jeweilige Position beibehalten und der Ultraschallwandler 59 mittels der Verstelleinheit 61 derart verstellt wird, daß seine Fokuszone bzw. der therapeutische Wirkbereich W innerhalb desjenigen Bereiches, in dem sich der von den Ultraschallwandlern 1 und 2 ausgehende Utraschall überlagert, in die zu behandelnden Gewebebereiche positioniert wird.

Zusätzlich ist in den Ultraschallwandler 1, der einen im Vergleich zur Fig. 1 vergrößerten Durchmesser aufweist, ein diagnostischer Ultraschall-Applikator 64 integriert, mittels dessen die für ein Ultraschall-Schnittbild einer den therapeutischen Wirkbereich W enthaltenden Körperschicht des Patienten erzeugbar ist. Der Ultraschall-Applikator 64, der in einer zentralen Bohrung des Ultraschallwandlers 1 aufgenommen ist, steht mit der Steuereinheit 34 über eine Steuer- und Signalleitung 65 in Verbindung. Die Steuereinheit 34 enthält die zur Ansteuerung des in dem Ultraschall-Applikator 64 enthaltenen Ultraschall-Transducers und zur Verarbeitung der von diesem gelieferten Signale sowie zur Erzeugung des Ultraschall-Schnittbildes erforderliche Elektronik. Das mit Hilfe des Ultraschall-Applikators 64 erzeugte Ultraschall-Schnittbild wird auf dem Monitor 49 angezeigt.

Das Ultraschall-Schnittbild kann zur Kontrolle des Therapie- Prozesses herangezogen werden. Außerdem besteht analog zur Ausführungsform gemäß Fig. 4 die Möglichkeit, die Ausrichtung des therapeutischen Wirkbereiches W unter Heranziehung des Ultraschall-Schnittbildes vorzunehmen.

Die Erfindung ist vorstehend am Beispiel der Behandlung der benignen Prostata-Hyperplasie beschrieben. Es können jedoch auch andere benigne oder maligne Gewebeveränderungen wie sonstige Hyperplasien oder Tumore behandelt werden.

Im Falle der beschriebenen Ausführungsbeispiele enthalten die Ultraschallwandler jeweils eine einzige scheibenförmige Piezokeramik. Ein anderer Aufbau der Ultraschallwandler ist möglich. Beispielsweise können diese eine mosaikartige Anordnung einer Vielzahl von piezokeramischen Wandlerelementen enthalten.

Die Fokussierung des mittels der Ultraschallwandler erzeugten Ultraschalls erfolgt im Falle der beschriebenen Ausführungsbeispiele jeweils mit Hilfe von akustischen Sammellinsen. Andere Arten der Fokussierung sind möglich, beispielsweise durch geeignete, insbesondere sphärischkonkave Formgebung oder durch elektronische Maßnahmen zur Fokussierung (phased array).

Die in den erfindungsgemäßen Therapieeinrichtungen enthaltenen Ultraschallwandler müssen nicht notwendigerweise mit der gleichen Frequenz betrieben werden. Es besteht vielmehr die Möglichkeit unterschiedliche Frequenzen zu wählen, was im Zusammenhang mit der Beeinflussung der Größe des jeweils vorhandenen therapeutischen Wirkbereiches von Vorteil sein kann. Aus dem gleichen Grunde kann es zweckmäßig sein, die einzelnen Ultraschallwandler derart anzusteuern, daß die jeweils erreichte maximale Ultraschallintensität für die einzelnen Ultraschallwandler unterschiedlich ist. Um hinsichtlich der Ausbreitung des von den einzelnen Ultraschallwandlern ausgehenden Ultraschalls optimale Ausbreitungsverhältnisse zu schaffen und/oder Schädigungen gesunden Gewebes vorzubeugen, kann es zweckmäßig sein, für die einzelnen Ultraschallwandler unterschiedliche Aperturwinkel vorzusehen. So ist es beispielsweise zweckmäßig, für den perineal applizierten Ultraschallwandler 2 einen relativ geringen Aperturwinkel vorzusehen, während für den nahe bei der Prostata 8 im Rektum 5 plazierten Ultraschallwandler 59 ein großer Aperturwinkel zweckmäßig ist.

Obwohl es meist zweckmäßig ist, mehrere therapeutische Ultraschallwandler vorzusehen, können erfindungsgemäße Therapieeinrichtungen mit nur einem einzigen therapeutischen Ultraschallwandler realisiert werden.

Unter Umständen kann es vorteilhaft sein, den zur Einführung in die Urethra bestimmten Katheter 35 und gegebenenfalls die im Rektum plazierte Meßsonde 57 mit mehr als zwei Druck- und/oder Temperatursensoren zu versehen. In diesem Fall besteht innerhalb gewisser Grenzen die Möglichkeit, in einem größeren Gewebebereich die Druck- bzw. Temperaturverteilung zu messen, was die Basis für eine exaktere Steuerung und Kontrolle des Therapieprozesses darstellt.

**Patentansprüche**

1. Therapieeinrichtung zur Behandlung von Gewebe (8) im Körper (3) eines Lebewesens, aufweisend wenigstens einen therapeutischen Ultraschallwandler (1, 2, 59), der einen therapeutischen Wirkbereich (W) aufweisenden Ultraschall aussendet, Mittel (29) zum Einkoppeln des mittels des Ultraschallwandlers (1, 2, 59) erzeugten Ultraschalls in den Körper (3) des Lebewesens, Mittel (22, 23, 32, 34, 61) zum Verlagern des therapeutischen Wirkbereiches (W) des Ultraschalls und des Körpers (3) des Lebewesens relativ zueinander, **dadurch gekennzeichnet, daß** Mittel (34, 35, 47, 48, 57) zur Messung der Ultraschallintensität, vorzugsweise der Amplitude des Ultraschalls vorgesehen sind, die unabhängig von der momentanen Lage des Wirkbereiches (W) die in dem Bereich des zu behandelnden Gewebes (8) vorliegende Ultraschallintensität messen, wobei die Verlagerung des therapeutischen Wirkbereiches (W) des Ultraschalls und des Körpers (3) des Lebewesens relativ zueinander anhand der mittels der Mittel (34, 35, 47, 48, 57) zur Messung der Ultraschallintensität gemessenen Ultraschallintensität erfolgt.

2. Therapieeinrichtung nach Anspruch 1, **dadurch gekennzeichnet, daß** die Mittel (34, 35, 47, 48, 57) zur Messung der Ultraschallintensität wenigstens ein Hydrophon (47, 48) enthalten.

3. Therapieeinrichtung nach Anspruch 1 oder 2, **dadurch gekennzeichnet, daß** Mittel (32, 34) zum Einstellen der im therapeutischen Wirkbereich (W) des Ultraschalls vorliegenden Ultraschallintensität vorgesehen sind.

4. Therapieeinrichtung nach einem der Ansprüche 1 bis 3, **dadurch gekennzeichnet, daß** Mittel (34, 35, 38, 39, 57) zum Messen der Temperatur im Bereich des zu behandelnden Gewebes (8) vorgesehen sind, welche wenigstens einen Temperatursensor (38, 39) aufweisen.

5. Therapieeinrichtung nach Anspruch 3 oder 4, **dadurch gekennzeichnet, daß** Mittel (34) zum Vergleichen der mittels der Mittel (34, 35, 47, 48, 57) zur Messung der Ultraschallintensität gemessenen Ultraschallintensität und/oder Mittel (34) zum Vergleichen der mittels der Mittel (34, 35, 38, 39, 57) zum Messen der Temperatur gemessenen Temperatur mit einem Sollwert vorgesehen sind.

6. Therapieeinrichtung nach einem der Ansprüche 1 bis 5, **dadurch gekennzeichnet, daß** Mittel (35, 44, 45, 46) zum Kühlen wenigstens eines an das zu behandelnden Gewebe (8) angrenzenden Gewebebereiches vorgesehen sind.

7. Therapieeinrichtung nach einem der Ansprüche 1 bis 6, **dadurch gekennzeichnet, daß** wenigstens zwei therapeutische Ultraschallwandler (1, 2, 59) vorgesehen sind, die Ultraschall jeweils in einer Hauptausbreitungsrichtung aussenden und die derart angeordnet sind, daß ihre Hauptausbreitungsrichtungen voneinander verschieden sind und daß sich der von ihnen ausgehende Ultraschall im Bereich des zu behandelnden Gewebes (8) überlagert.

8. Therapieeinrichtung nach Anspruch 7, **dadurch gekennzeichnet,** daß eine Steuereinheit (34) vorgesehen ist, die die Ultraschallwandler (1, 2, 59) derart antreibt, daß die Ultraschallwandler (1, 2, 59) jeweils Ultraschall solcher Ultraschallintensität abstrahlen, daß sich der therapeutische Wirkbereich (W) innerhalb desjenigen Bereiches befindet, in dem sich der Ultraschall überlagert.

9. Therapieeinrichtung nach einem der Ansprüche 7 oder 8, **dadurch gekennzeichnet,** daß die Ultraschallwandler (1, 2, 59) den Ultraschall fokussiert abstrahlen und daß die jeweilige Fokuszonen im Bereich der Überlagerung des Ultraschalls liegen.

10. Therapieeinrichtung nach Anspruch 9, **dadurch gekennzeichnet,** daß die Fokuszonen einander zumindest teilweise überlappen.

11. Therapieeinrichtung nach Anspruch 9 oder 10, **dadurch gekennzeichnet,** daß die Fokuszonen unterschiedliche Abmessungen aufweisen und/oder von unterschiedlicher Gestalt sind.

12. Therapieeinrichtung nach Anspruch 11, **dadurch gekennzeichnet,** daß eine kleinere und eine größere Fokuszone vorgesehen sind und daß die Mittel (22, 23, 32, 34, 61) zum Verlagern des therapeutischen Wirkbereiches (W) den Wirkbereich (W) und den Körper (3) des Lebewesens relativ zueinander in der Weise verlagern, daß sie die kleinere Fokuszone innerhalb der größeren Fokuszone verlagern.

13. Therapieeinrichtung nach einem der Ansprüche 7 bis 12, **dadurch gekennzeichnet,** daß der Wirkbereich (W) im wesentlichen von sphärischer Gestalt ist.

14. Therapieeinrichtung nach einem der Ansprüche 7 bis 13, **dadurch gekennzeichnet,** daß eine Steuereinheit (34) vorgesehen ist, die die Ultraschallwandler (1, 2, 59) derart antreibt, daß die Ultraschallwandler (1, 2, 59) Ultraschall unterschiedlicher Frequenz abstrahlen.

15. Therapieeinrichtung nach einem der Ansprüche 7 bis 14, **dadurch gekennzeichnet,** daß eine Steuereinheit (34) vorgesehen ist, die die Ultraschallwandler (1, 2, 59) derart antreibt, daß die Amplituden des von den Ultraschallwandlern (1, 2, 59) jeweils abgestrahlten Ultraschalls im Bereich der Überlagerung des Ultraschalls unterschiedlich sind.

16. Therapieeinrichtung nach einem der Ansprüche 7 bis 15, **dadurch gekennzeichnet,** daß die Aperturwinkel der Ultraschallwandler (1, 2, 59) unterschiedlich sind.

17. Therapieeinrichtung nach einem der Ansprüche 7 bis 16, **dadurch gekennzeichnet,** daß die Ultraschallwandler (1, 2, 59) unterschiedliche Fokussierungsgrade aufweisen.

18. Therapieeinrichtung nach einem der Ansprüche 1 bis 17, **dadurch gekennzeichnet,** daß jeder der Ultraschallwandler (1, 2, 59) mittels der Mittel (22, 23, 32, 34, 61) zum Verlagern um zwei einander vorzugsweise rechtwinklig schneidende Achsen (A, B) schwenkbar ist.

19. Therapieeinrichtung nach einem der Ansprüche 1 bis 18, **dadurch gekennzeichnet,** daß der therapeutische Ultraschallwandler (59) in einer für die rektale Applikation vorgesehenen Sonde (60) aufgenommen ist.

20. Therapieeinrichtung nach Anspruch 19, **dadurch gekennzeichnet,** daß der Ultraschallwandler (59) mittels der Mittel (32, 34, 61) zum Verlagern um die Längsachse der Sonde (60) verdrehbar und um eine die Längsachse der Sonde (60) vorzugsweise rechtwinklig schneidende Achse schwenkbar ist.

21. Therapieeinrichtung nach einem der Ansprüche 1 bis 20, **dadurch gekennzeichnet,** daß die Therapieeinrichtung zur Behandlung der benignen Prostatahyperplasie vorgesehen ist und daß die Mittel (34, 35, 47, 48, 57) zur Messung der Intensität des Ultraschalls und/ oder die Mittel (34, 35, 38, 39, 57) zur Messung der Temperatur und/oder die Mittel zum Kühlen (35, 44, 45, 46) in einen in die Urethra einführbaren Katheter (35) und/oder in eine in das Rektum (5) einführbare Meßsonde (57) integriert sind.

22. Therapieeinrichtung nach einem der Ansprüche 1 bis 21, **dadurch gekennzeichnet,** daß jeder der Ultraschallwandler (1, 2, 59) zur Aussendung eines Pilotsignals verminderter Intensität ansteuerbar ist, und daß die Ausrichtung des Ultraschallwandlers (1, 2, 59) und des Körpers (3) des Lebewesens relativ zueinander anhand des Pilotsignals erfolgt.

23. Therapieeinrichtung nach Anspruch 22, **dadurch gekennzeichnet,** daß jeweils nur ein Ultraschallwandler (1, 2, 59) gleichzeitig zur Aussteuerung des Pilotsignals ansteuerbar ist.

24. Therapieeinrichtung nach einem der Ansprüche 1 bis 23, **dadurch gekennzeichnet,** daß eine für die rektale Applikation geeignete Ultraschall-Diagnostik-Sonde (52) vorgesehen ist, mit Hilfe derer Ultraschall-Schnittbilder zweier einander schneidender, den Bereich des zu behandelnden Gewebes (8) enthaltender Körperschichten (X, Y) des Lebewesens erzeugbar sind, und daß die Verlagerung des therapeutischen Wirkbereiches (W) des Ultraschalls und des Körpers (3) des Lebewesens relativ zueinander unter Berücksichtigung der mit Hilfe der Ultraschall-Diagnostik-Sonde (52) erzeugten Ultraschall-Schnittbilder erfolgt.

25. Therapieeinrichtung nach einem der Ansprüche 1 bis 24, **dadurch gekennzeichnet,** daß in wenigstens einen Ultraschallwandler (1) ein diagnostischer Ultraschall-Applikator (64) integriert ist, mit dessen Hilfe ein Ultraschall-Schnittbild einer den therapeutischen Wirkbereich (W) des Ultraschalls enthaltenden Körperschicht des Lebewesens erzeugbar ist, und daß die Verlagerung des therapeutischen Wirkbereiches (W) des Ultraschalls und des Körpers des Lebewesens relativ zueinander unter Berücksichtigung des mit Hilfe des diagnostischen Ultraschall-Applikators (64) erzeugten Ultraschall-Schnittbildes erfolgt.

## Claims

1. Therapy apparatus for the treatment of tissue (8) in the body (3) of a life form, exhibiting at least one therapeutic ultrasound transducer (1, 2, 59) that emits an ultrasound having a therapeutic effective region (W), means (29) for coupling the ultrasound generated with the ultrasound transducer (1, 2, 59) into the body (3) of the life form, means (22, 23, 32, 34, 61) for displacing the therapeutic effective region (W) of the ultrasound and of the body (3) of life form relative to one another, characterised in that means (34, 35, 47, 48, 57) for measuring the ultrasound intensity, preferably the amplitude of the ultrasound are provided, which independently of the momentary position of the effective region (W) measure the ultrasound intensity present in the region of the tissue (8) to be treated, the displacement of the therapeutic effective region (W) of the ultrasound and of the body (3) of the life form relative to one another being effected using the ultrasound intensity measured using the means (34, 35, 47, 48, 57) for measuring the ultrasound intensity.

2. Therapy apparatus according to Claim 1,
characterised in that the means (34, 35, 47, 48, 57) for measuring the ultrasound intensity include at least one hydrophone (47, 48).

3. Therapy apparatus according to Claim 1 or 2,
characterised in that means (32, 34) are provided for setting the ultrasound intensity present in the effective therapeutic region (W) of the ultrasound.

4. Therapy apparatus according to one of Claims 1 to 3, characterised in that means (34, 35, 38, 39, 57) for measuring the temperature in the region of the tissue (8) to be treated are provided having at least one temperature sensor (38, 39).

5. Therapy apparatus according to Claim 3 or 4,
characterised in that means (34) for comparing the ultrasound intensity measured using the means (34, 35, 47, 48, 57) for measuring the ultrasound intensity and/or means (34) for comparing the temperature measured with the means (34, 35, 38, 39, 57) for measuring the temperature are provided with a reference value.

6. Therapy apparatus according to one of the Claims 1 to 5, characterised in that means (35, 44, 45, 46) are provided for cooling at least one of the regions of tissue adjoining the tissue (8) to be treated.

7. Therapy apparatus according to one of Claims 1 to 6, characterised in that at least two therapeutic ultrasound transducers (1, 2, 59) are provided, which emit ultrasound respectively in a principal direction of propagation and which are arranged such that their principal directions of propagation vary from one another and in that the ultrasound emanating from them is superimposed in the region of the tissue (8) to be treated.

8. Therapy apparatus according to Claim 7,

characterised in that a control unit (34) is provided which drives the ultrasound transducers (1, 2, 59) such that the ultrasound transducers (1, 2, 59) emit respectively ultrasound of such ultrasonic intensity that the therapeutic effective region (W) is located within that region in which the ultrasound is superimposed.

9. Therapy apparatus according to one of the Claims 7 or 8, characterised in that the ultrasound transducers (1, 2, 59) emit the ultrasound in a focused fashion and in that the respective focus zones are located within the region of superimposition of the ultrasound.

10. Therapy apparatus according to Claim 9,
characterised in that the focus zones overlap each other at least to some extent.

11. Therapy apparatus according to Claim 9 or 10,
characterised in that the focus zones have various dimensions and/or varying shape.

12. Therapy apparatus according to Claim 11,
characterised in that a smaller and a larger focus zone are provided and in that the means (22, 23, 32, 34, 61) for displacing the therapeutic effective region (W) displace the effective region (W) and the body (3) of the life form relative to one another such that they displace the smaller focus zone within the larger focus zone.

13. Therapy apparatus according to one of the Claims 7 to 12, characterised in that the effective region (W) is essentially of spherical shape.

14. Therapy apparatus according to one of the Claims 7 to 13, characterised in that a control unit (34) is provided which drives the ultrasound transducers (1, 2, 59) in such a way that the ultrasound transducers (1, 2, 59) emit ultrasound of varying frequency.

15. Therapy apparatus according to one of the Claims 7 to 14, characterised in that a control unit (34) is provided which drives the ultrasound transducers (1, 2, 59) in such a way that the amplitudes of the ultrasound emitted respectively from the ultrasound transducers (1, 2, 59) vary in the region of superimposition of the ultrasound.

16. Therapy apparatus according to one of the Claims 7 to 15, characterised in that the aperture angles of the ultrasound transducers (1, 2, 59) vary.

17. Therapy apparatus according to one of the Claims 7 to 16, characterised in that the ultrasound transducers (1, 2, 59) have various degrees of focusing.

18. Therapy apparatus according to one of Claims 1 to 17, characterised in that each of the ultrasound transducers (1, 2, 59) can be pivoted with the means (22, 23, 32, 34, 61) for displacing around two axes (A, B) which intersect one another preferably at right angles.

19. Therapy apparatus according to one of the Claims 1 to 18, characterised in that the therapeutic ultrasound transducer (59) is accepted in a probe (60) provided for rectal application.

20. Therapy apparatus according to Claim 19,
characterised in that the ultrasound transducer (59) is rotatable around the longitudinal axis of the probe (60) using the means (32, 34, 61) for displacement and is pivotable around an axis which intersects the longitudinal axis of the probe (60) preferably at right angles.

21. Therapy apparatus according to one of the Claims 1 to 20, characterised in that the therapy apparatus is provided for treating benign prostate hyperplasia and in that the means (34, 35, 47, 48, 57) for measuring the intensity of the ultrasound and/or the means (34, 35, 38, 39, 57) for measuring the temperature and/or the means (35, 44, 45, 46) for cooling are integrated in a catheter (35) that can be introduced into the urethra and/or in a measuring probe (57) that can be introduced into the rectum (5).

22. Therapy apparatus according to one of the Claims 1 to 21, characterised in that each of the ultrasound transducers (1, 2, 59) for outputting a pilot signal of reduced intensity can be controlled, and in that the alignment of the ultrasound transducer (1, 2, 59) and of the body (3) of the life form are aligned relative to one another on the basis of the pilot signal.

**23.** Therapy apparatus according to Claim 22,
characterised in that only one ultrasound transducer (1, 2, 59) respectively can be controlled at a time for modulating the pilot signal.

**24.** Therapy apparatus according to one of the Claims 1 to 23, characterised in that an ultrasound diagnostics probe (52) suitable for rectal application is provided, with the aid of which ultrasound tomograms of two body slices (X, Y) of the life form which intersect one another and contain the region of the tissue to be treated (8) can be generated, and in that the displacement of the therapeutic effective region (W) of the ultrasound and of the body (3) of the life form relative to one another is effected taking into account the ultrasound tomograms generated with the aid of the ultrasound diagnostics probe (52).

**25.** Therapy apparatus according to one of the Claims 1 to 24, characterised in that in at least one ultrasound transducer (1) a diagnostic ultrasound applicator (64) is integrated with the aid of which an ultrasound tomogram of a body slice of the life form containing the therapeutic effective region (W) of the ultrasound can be generated, and in that the displacement of the therapeutic effective region (W) of the ultrasound and of the body of the life form relative to one another is effected taking into account the ultrasound tomogram generated with the aid of the diagnostics ultrasound applicator (64).

## Revendications

**1.** Dispositif de thérapie pour le traitement de tissus (8) du corps (3) d'un être vivant, comportant au moins un transducteur (1, 2, 59) à ultrasons de thérapie qui émet un ultrason ayant une région (W) active de thérapie, des moyens (29) servant à envoyer dans le corps (3) de l'être vivant l'ultrason produit au moyen du transducteur (1, 2, 59) à ultrasons, des moyens (22, 23, 32, 34, 61) servant à déplacer l'une par rapport à l'autre la région (W) active de thérapie de l'ultrason et le corps (3) de l'être vivant, caractérisé en ce qu'il est prévu des moyens (34, 35, 47, 48, 57) pour mesurer l'intensité de l'ultrason, de préférence l'amplitude de l'ultrason, qui mesurent l'intensité d'ultrason présente dans la zone du tissu (8) à traiter indépendamment de la position sur le moment de la région (W) active, le déplacement l'une par rapport à l'autre de la région (W) active de thérapie de l'ultrason et du corps (3) de l'être vivant s'effectuant à l'aide de l'intensité d'ultrason mesurée à l'aide des moyens (34, 35, 47, 48, 57) de mesure de l'intensité de l'ultrason.

**2.** Dispositif de thérapie suivant la revendication 1, caractérisé en ce que les moyens (34, 35, 47, 48, 57) de mesure de l'intensité de l'ultrason comportent au moins un hydrophone (47, 48).

**3.** Dispositif de thérapie suivant la revendication 1 ou 2, caractérisé en ce qu'il est prévu des moyens (32, 34) pour régler l'intensité d'ultrason présente dans la région (W) active de thérapie de l'ultrason.

**4.** Dispositif de thérapie suivant l'une des revendications 1 à 3, caractérisé en ce qu'il est prévu des moyens (34, 35, 38, 39, 57) pour repérer la température dans la zone du tissu (8) à traiter, qui comportent au moins un capteur (38, 39) de température.

**5.** Dispositif de thérapie suivant la revendication 3 ou 4, caractérisé en ce qu'il est prévu des moyens (34) pour comparer à une valeur de consigne l'intensité d'ultrason mesurée au moyen des moyens (34, 35, 47, 48, 57) de mesure de l'intensité de l'ultrason et/ou des moyens (34) pour comparer à une valeur de consigne la température repérée au moyen des moyens (34, 35, 38, 39, 57) de mesure de la température.

**6.** Dispositif de thérapie suivant l'une des revendications 1 à 5, caractérisé en ce qu'il est prévu des moyens (35, 44, 45, 46) pour refroidir au moins une zone de tissu contiguë au tissu (8) à traiter.

**7.** Dispositif de thérapie suivant l'une des revendications 1 à 6, caractérisé en ce qu'il est prévu au moins deux transducteurs (1, 2, 59) de thérapie à ultrasons, qui émettent chacun un ultrason dans une direction principale de propagation et qui sont disposés de manière que leurs directions principales de propagation soient différentes l'une de l'autre et que l'ultrason qui part de ces transducteurs se superpose dans la zone du tissu (8) à traiter.

**8.** Dispositif de thérapie suivant la revendication 7, caractérisé en ce qu'il est prévu une unité (34) de commande, qui entraîne les transducteurs (1, 2, 59) à ultrasons de manière que les transducteurs (1, 2, 59) à ultrasons émettent chacun un ultrason d'une intensité d'ultrason telle que la région (W) active de thérapie se trouve dans le région dans laquelle l'ultrason se superpose.

9. Dispositif de thérapie suivant l'une des revendications 7 ou 8, caractérisé en ce que les transducteurs (1, 2, 59) à ultrasons émettent l'ultrason de manière focalisée et en ce que les zones focales associées se trouvent dans la zone de la superposition de l'ultrason.

10. Dispositif de thérapie suivant la revendication 9, caractérisé en ce que les zones focales se chevauchent l'une l'autre au moins partiellement.

11. Dispositif de thérapie suivant la revendication 9 ou 10, caractérisé en ce que les zones focales ont des dimensions différentes et/ou sont de forme différente.

12. Dispositif de thérapie suivant la revendication 11, caractérisé en ce qu'il est prévu une zone focale plus petite et une zone focale plus grande et en ce que les moyens (22, 23, 32, 34, 61) servant à déplacer la région (W) active de thérapie déplacent l'une par rapport à l'autre la région (W) active et le corps (3) de l'être vivant de manière à déplacer la zone focale plus petite à l'intérieur de la zone focale plus grande.

13. Dispositif de thérapie suivant l'une des revendications 7 à 12, caractérisé en ce que la région (W) active est de forme sensiblement sphérique.

14. Dispositif de thérapie suivant l'une des revendications 7 à 13, caractérisé en ce qu'il est prévu une unité (34) de commande, qui entraîne les transducteurs (1, 2, 59) à ultrasons de manière que les transducteurs (1, 2, 59) à ultrasons émettent un ultrason de fréquence différente.

15. Dispositif de thérapie suivant l'une des revendications 7 à 14, caractérisé en ce qu'il est prévu une unité (34) de commande, qui entraîne les transducteurs (1, 2, 59) à ultrasons de manière que les amplitudes de l'ultrason émis par chacun des transducteurs (1, 2, 59) à ultrasons soient différentes dans la région de la superposition de l'ultrason.

16. Dispositif de thérapie suivant l'une des revendications 7 à 15, caractérisé en ce que les angles d'ouverture des transducteurs (1, 2, 59) à ultrasons sont différents.

17. Dispositif de thérapie suivant l'une des revendications 7 à 16, caractérisé en ce que les transducteurs (1, 2, 59) à ultrasons ont des degrés différents de focalisation.

18. Dispositif de thérapie suivant l'une des revendications 1 à 17, caractérisé en ce que chacun des transducteurs (1, 2, 59) à ultrasons peut être basculé à l'aide des moyens (22, 23, 32, 34, 61) de déplacement par rapport à deux axes (A, B) qui se coupent de préférence à angle droit.

19. Dispositif de thérapie suivant l'une des revendications 1 à 18, caractérisé en ce que le transducteur (59) à ultrasons de thérapie est logé dans une sonde (60) prévue pour l'application rectale.

20. Dispositif de thérapie suivant la revendications 19, caractérisé en ce que, à l'aide des moyens (32, 34, 61) de déplacement, le transducteur (59) à ultrasons peut être tourné par rapport à l'axe longitudinal de la sonde (60) et peut être basculé par rapport à un axe coupant l'axe longitudinal de la sonde (60) à angle droit.

21. Dispositif de thérapie suivant l'une des revendications 1 à 20, caractérisé en ce que le dispositif de thérapie est prévu pour le traitement de l'hyperplasie bénigne de la prostate et en ce que les moyens (34, 35, 47, 48, 57) de mesure de l'intensité de l'ultrason et/ou les moyens (34, 35, 38, 39, 57) de repérage de la température et/ou les moyens de refroidissement (35, 44, 45, 46) sont intégrés à un cathéter (35) pouvant être introduit dans l'urètre et/ou à une sonde (57) de mesure pouvant être introduite dans le rectum.

22. Dispositif de thérapie suivant l'une des revendications 1 à 21, caractérisé en ce que chacun des transducteurs (1, 2, 59)) à ultrasons peut être commandé pour émettre un signal pilote d'intensité réduite et en ce que l'alignement l'un part rapport à l'autre du transducteur (1, 2, 59) à ultrasons et du corps (3) de l'être vivant s'effectue à l'aide du signal pilote.

23. Dispositif de thérapie suivant la revendications 22, caractérisé en ce qu'un seul transducteur (1, 2, 59) à ultrasons peut être commandé en même temps pour régler le signal pilote.

24. Dispositif de thérapie suivant l'une des revendications 1 à 23, caractérisé en ce qu'il est prévu une sonde (52) de diagnostic à ultrasons, qui convient à l'application rectale et à l'aide de laquelle on peut produire des images en coupe à ultrasons de deux couches (X, Y) corporelles de l'être vivant se coupant l'une l'autre et contenant la région du tissu (8) à traiter et en ce que le déplacement l'une par rapport à l'autre de la région (W) active de thérapie de l'ultrason et du corps (3) de l'être vivant s'effectue en tenant compte des images en coupe à ultrasons produites à l'aide de la sonde (52) de diagnostic à ultrasons.

25. Dispositif de thérapie suivant l'une des revendications 1 à 24, caractérisé en ce qu'il est intégré à au moins un transducteur (1) à ultrasons un applicateur (64) de diagnostic à ultrasons, à l'aide duquel peut être produite une image en coupe à ultrasons d'une couche corporelle de l'être vivant contenant la région (W) active de thérapie de l'ultrason, et en ce que le déplacement l'une par rapport à l'autre de la région (W) active de thérapie de l'ultrason et du corps (3) de l'être vivant s'effectue en tenant compte des images en coupe à ultrasons produites à l'aide de l'applicateur (64) de diagnostic à ultrasons.

FIG 1

FIG 2

FIG 3

FIG 4

EP 0 630 220 B1

FIG 5